# EUROPEAN PATENT APPLICATION

(11) **EP 4 529 874 A1**
(43) Date of publication of application: **02.04.2025**
(21) Application number: 24203077.3
(22) Date of filing: 27.09.2024
(51) Int. Cl.: A61B 18/14, A61B 34/10, A61B 34/20

(54) **EPICARDIAL ABLATION AND MAPPING CATHETER**

(30) Priority: 28.09.2023 US 202318374137
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); SITNITSKY, Ilya, 2066717 Yokneam (IL); SHECHTMAN, Alexander, 2066717 Yokneam (IL)
(74) Representative: Carpmaels & Ransford LLP

(57) **Abstract**

A catheter for mapping and ablation of epicardial tissue, including: a shaft configured for insertion into a body of a subject; a distal assembly configured at a distal end of the shaft, the distal assembly being expandable from a collapsed state to a deployed state; the distal assembly comprising at least two frame elements; each frame element comprising an arched distal portion on which a plurality of electrodes for delivery of ablation energy are disposed; wherein in the deployed state, the distal assembly assumes a concave profile for conforming to a curvature of the epicardium; and a plurality of position sensors disposed on one or both of the shaft and the frame elements for tracking a position and an orientation of at least a portion of the distal assembly or the shaft.

## Description

### TECHNOLOGICAL FIELD

This invention relates to cardiac catheters, and, in particular, to a catheter for mapping and/or ablation of epicardial tissue.

### BACKGROUND

US10966623B2 discloses "A catheter adapted or high-density mapping and/or ablation of tissue surface has a distal electrode array with offset spine loops, each spine loop having at least a pair of linear portions and a distal portion connecting the pair of linear portions, and one or more electrodes on each linear portion. The linear portions of the plurality of offset spine loops are arranged in-plane a single common plane, and the distal portions of the plurality of offset spine loops are arranged off-plane from the single common plane".

### BRIEF DESCRIPTION OF THE DRAWINGS

In order to better understand the subject matter that is disclosed herein and to exemplify how it may be carried out in practice, embodiments will now be described, by way of non-limiting examples only, with reference to the accompanying drawings, in which:
**FIG. 1** illustrates a catheter-based electrophysiology mapping and ablation system, according to examples of the presently disclosed subject matter;
**FIG. 2** is a flowchart of a general method for ablating and/or mapping epicardial tissue, according to examples of the presently disclosed subject matter;
**FIGs. 3A-C**are schematic top views (FIG. 3A, FIG. 3C) and a schematic side cross section view (FIG. 3B) of a distal tip of a catheter for ablation and/or mapping of epicardial tissue, according to examples of the presently disclosed subject matter;
**FIG. 4** is a schematic illustration of a portion of the distal tip of the catheter shown in FIGs. 3A-B, placed in contact with epicardial tissue, according to examples of the presently disclosed subject matter; and
**FIG. 5** is a schematic cross section view of the distal tip of the catheter shown in FIGs. 3A-C, showing the distal assembly in the collapsed state inside a sheath of the catheter, according to examples of the presently disclosed subject matter.

### DETAILED DESCRIPTION OF EXAMPLES

In the following detailed description, numerous specific details are set forth in order to provide a thorough understanding of the invention. However, it will be understood by those skilled in the art that the presently disclosed subject matter may be practiced without these specific details. In other instances, well-known methods, procedures, components and circuits have not been described in detail so as not to obscure the presently disclosed subject matter.

Unless specifically stated otherwise, as apparent from the following discussions, it is appreciated that throughout the specification discussions utilizing terms such as "processing", "computing", "comparing", or the like, refer to the action(s) and/or process(es) of a computer that manipulate and/or transform data into other data, said data represented as physical, such as electronic, quantities and/or said data representing the physical objects. The term "computer" should be expansively construed to cover any kind of hardware-based electronic device with data processing capabilities including, by way of non-limiting example, a Fabrication Process Examination Information (FPEI) system and respective parts thereof disclosed in the present application.

The present disclosure relates to electrophysiology mapping and/or ablation of the epicardium of the heart, performed using a catheter. One challenge involved in such procedure is how to effectively and efficiently ablate a large tissue surface area. Another challenge is how to enhance the contact of the electrodes with the tissue, for example for ensuring the transfer of ablation energy to the tissue. Another challenge is how to engage the tissue in an anatomy which is naturally curved or rounded, e.g. convex. Yet another challenge is how to introduce and position the catheter in the limited volume of the pericardial cavity, and to treat from within that cavity.

One or more of the above-described challenges may be overcome using a catheter device for example as described herein:
According to a first aspect, the catheter comprises a distal assembly on which a plurality of electrodes are mounted, for ablating and/or position sensing, and the distal assembly is configured to assume a concave profile (at least at a portion thereof) shaped to conform to the rounded tissue surface (e.g. a convex surface) of the epicardium. In use, the distal assembly (at the concave portion thereof) spoons the tissue, thereby potentially increasing the contact area between the electrodes and the tissue, for example as compared to a planar (flat) distal assembly.

According to a second aspect, one or more inflatable elements (e.g., a balloon) can be positioned with respect to a back side of the distal assembly (i.e., a side opposing that which contacts the epicardium tissue). The inflatable element can be effective to one or more of: support and stabilize the electrodes against the epicardium tissue, for example during ablation; provide insulation to the back side of the electrodes; form or deepen the concavity of the distal assembly, as the inflatable element presses against the distal assembly when inflated within the limited volume of the pericardium cavity.

According to a third aspect, the distal assembly is manipulable between a collapsed state, in which it is compactly fitted within a sheath of the catheter; and a deployed, expanded state, in which the distal assembly assumes the concave (C-shaped) profile (at least at a portion thereof) for being placed in contact with the tissue. In some examples, the distal assembly comprises two or more loop-like frame elements which at least partially overlap each other, each of the frame elements defining a closed contour with the catheter shaft. In the collapsed state of the assembly, each of the frame elements is compressed (for example, with respect to its longitudinal axis), facilitating the receipt of the distal assembly inside the sheath; in the deployed, expanded state of the assembly, each of the frame elements expands laterally with respect to its longitudinal axis, and an outer contour of the two frame elements together thereby encompasses a large tissue surface area.

In some examples, the electrodes are arranged on the frame elements in a staggered manner. A staggered electrode arrangement may be advantageous in that the contact areas of the electrodes with the tissue can be densely distributed and thereby provide increased coverage of the tissue being ablated; another potential advantage of a staggered arrangement is that in the collapsed state of the distal assembly, the electrodes fit in an alternating manner, thus enabling the distal assembly to fit within a small diameter of the sheath.

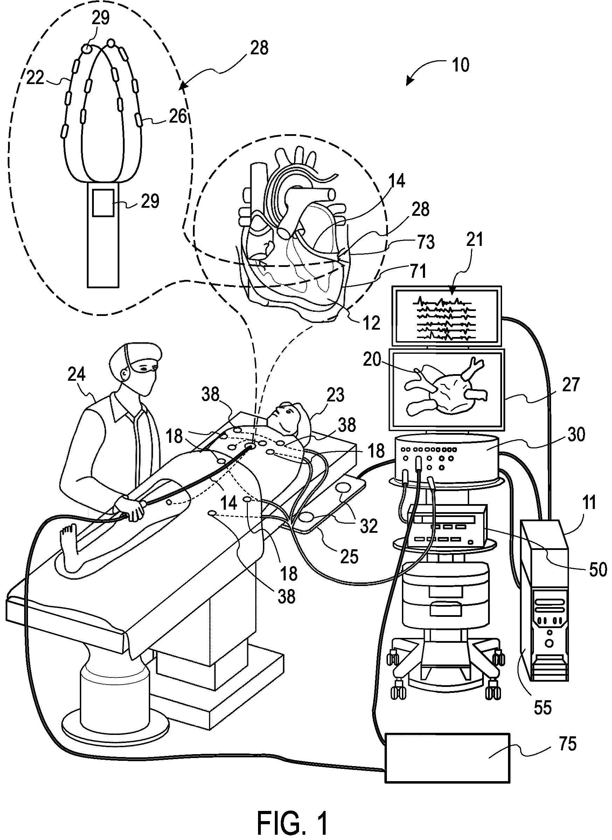

Reference is made to **FIG. 1** showing an example catheter-based electrophysiology mapping and ablation system 10. System 10 includes at least one catheter, which is inserted by a physician 24 to the heart 12. Typically, a delivery sheath is inserted in an intrathoracic approach and guided to a desired location in heart 12, optionally via the apex of the heart. Thereafter, the catheter may be inserted into the delivery sheath catheter so as to arrive at the desired location in heart 12. In some examples, the desired location is the epicardium 71, and the catheter is introduced through the pericardium 73 so as to reach the epicardium.

An example catheter 14 that is configured for ablating tissue and/or for sensing electrical cardiac activity and/or mapping is illustrated herein. Physician 24 may place a distal tip 28 of catheter 14 in contact with the epicardial tissue surface for sensing and/or ablating.

Catheter 14 is an exemplary catheter that includes a distal assembly, having one and preferably multiple electrodes 26 optionally distributed over a plurality of frame elements 22 at distal tip 28. The electrodes are generally configured for delivering ablation energy to tissue, and/or for sensing cardiac electrical signals. Catheter 14 additionally includes one or more position sensors 29 embedded in or near distal tip 28 for tracking position and orientation of distal tip 28. Optionally and preferably, position sensor 29 is a magnetic based position sensor, for example a position sensor including three magnetic coils for sensing three-dimensional (3D) position and orientation; or a position sensor including one magnetic coil, for sensing a single direction.

Each of the magnetic based position sensors 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location reference for location pad 25 as well as impedance-based tracking of electrodes 26. For impedance-based tracking, electrical current is directed to electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 records and displays electrograms 21 captured with body surface ECG electrodes 18 and optionally cardiac signals captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to electrodes 26 of the distal assembly at a distal tip of the catheter. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between the at least one catheter with other catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software stored therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of the at least one catheter within the heart chamber, and (4) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

In some examples, an irrigation module 75 is provided for delivering irrigation fluid, such as saline solution, to the location of treatment. The irrigation module may comprise of a pump and an associated fluid tank.

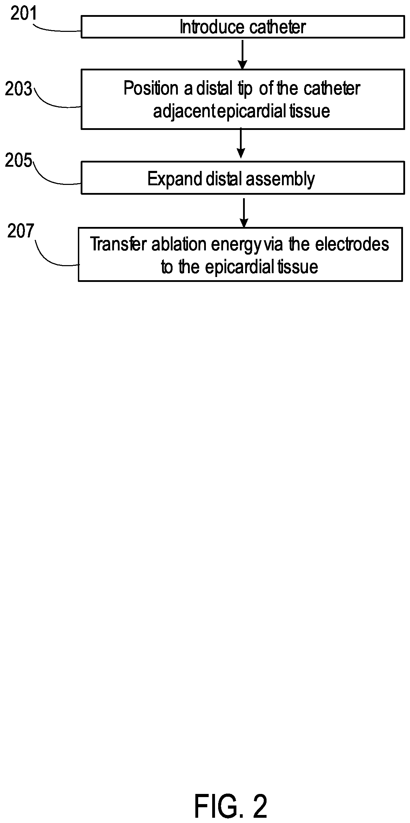

**FIG. 2** is a flowchart of a general method for ablating and/or mapping epicardial tissue, according to examples of the presently disclosed subject matter.

At 201, a catheter is introduced into the patient's body. In some cases, the catheter is introduced in a transthoracic approach. Then, the catheter is guided under imaging guidance towards the heart, and a distal tip of the catheter is advanced to the cardiac location intended for treatment. At 203, the distal tip is inserted through the pericardium (optionally, from the direction of the apex) and is then positioned adjacent epicardial tissue.

In some examples, optionally, using position sensor(s) of the catheter (e.g., position sensors including magnetic coil(s), for example as described herein) a position and/or orientation of the distal tip of the catheter or portions thereof is sensed. Optionally, location coordinates are generated.

In some examples, mapping of the surrounding tissues is performed based on data from the position sensors, such as by acquiring data from a multiplicity of points. In some examples, data from the position sensors is used for determining trajectory information, from which motion characteristics such as the contractility of the tissue can be inferred.

At 205, according to some examples, a distal assembly at a distal tip of the catheter is expanded. In some examples, during insertion of the catheter the distal assembly is maintained in a collapsed state in which the assembly is contained inside a sheath of the catheter; and when the distal tip is brought adjacent the target location (e.g., an epicardial site), the assembly is expanded. In some examples, the distal assembly, when expanded, assumes a spoon-like curvature which is concave towards the tissue to be treated, as will be further described herein. In the expanded state, electrodes mounted on the distal assembly can be placed in contact with the tissue.

In some examples, an inflatable element such as a balloon is inflated to push the electrodes against the tissue, and stabilize the electrodes in position. In some cases, the inflatable element is advanced from the catheter sheath, and is then filled with fluid (e.g., saline) to apply pressure onto the distal assembly from one side thereof. In some cases, the inflatable element is positioned in the pericardial cavity, between the distal assembly and the parietal layer of the pericardium, such that the parietal layer of the pericardium provides a counter force acting on the inflatable element.

In some examples, cardiac electrical signals are sensed, for example using one or more electrodes of the distal assembly. In some cases, electrodes used for sensing electrical signals are the same as the ablation electrodes. Additionally or alternatively, separate electrodes which are dedicated to sensing electrical signals are used. In some examples, a location of the sensed signals is assessed with the aid of the one or more position sensors mounted on the catheter. Optionally, cardiac activity patterns are determined, for example to identify arrythmias (e.g., tachycardia, such as ventricular tachycardia) or other disorders and optionally their source, for example, to identify scarring on the epicardium which is associated with the abnormal cardiac signals. In this manner, the location(s) intended for ablation can be carefully identified and selected.

At 207, according to some examples, ablation energy (e.g., higher power energy such as RF, PFA,) is transferred via at least some of the electrodes of the distal assembly to the epicardial tissue. Holding the electrodes stable and in close contact with the tissue, such as with the aid of the inflatable element, may be advantageous for maximizing a contact surface area of the electrodes with the tissue and potentially improving the delivery of ablation energy into the tissue.

In some cases, the distal assembly is steered towards one or more additional target locations (optionally, of the epicardium) for performing ablation. The target locations may be selected based on the sensed and/or mapped electrical cardiac activity, so as to block or modify an arrhythmogenic electrical pathway.

In some examples, irrigation is performed. In some cases, irrigation is performed to cool the tissue, for example so as to reduce collateral damage to tissues adjacent the target site. Irrigation may further cool the electrodes, for example the energy emitting surfaces of the electrodes, thereby potentially preventing overheating of the electrodes. Various irrigation methods will be described hereinbelow.

It is noted that position sensing, cardiac electrical activity sensing, and/or mapping of the tissue or anatomy may be performed throughout the procedure or at any required times during the procedure.

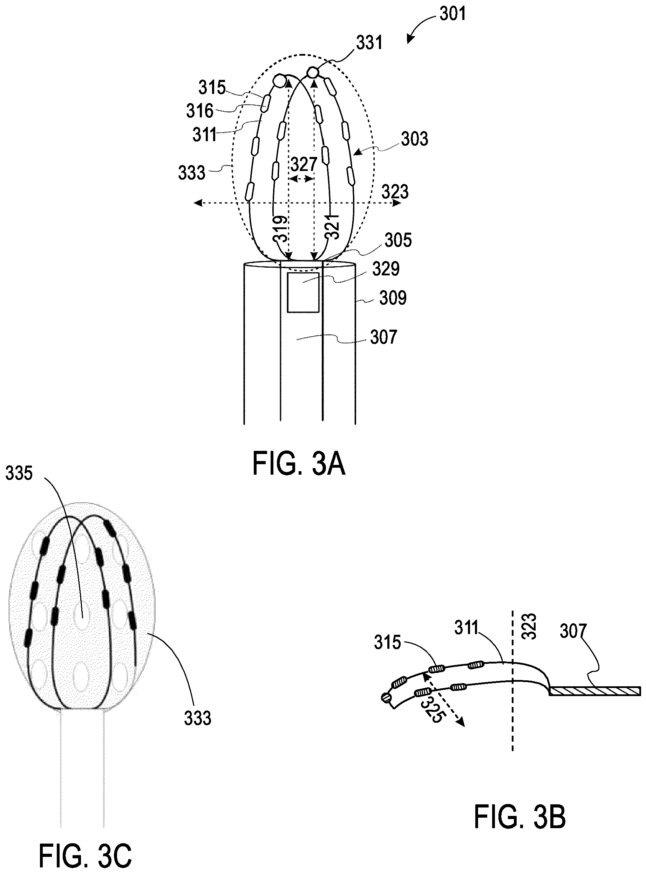

**FIGs. 3A-C**are schematic top views (3A, 3C) and a schematic side cross section view (3B) of a distal tip of a catheter for ablation and/or mapping of epicardial tissue, according to examples of the presently disclosed subject matter.

In some examples, a distal tip 301 of the catheter comprises a distal assembly 303 extending from a distal end 305 of a shaft 307.

The distal assembly is configurable between two states: a first state in which the assembly is collapsed so as to be contained within a sheath 309 of the catheter; and a second state, shown herein, in which the assembly is deployed, protruding outwardly from the distal end of the sheath.

The distal assembly, in accordance with this example, comprises at least two frame elements 311, e.g., looped splines, each defining a closed contour with the distal end of the shaft. The two frame elements at least partially overlap each other. Each of the frame elements comprises an arched distal portion, for example, a parabolic distal portion. In some examples, the frame element has a generally elongate loop shape, for example, oval, elliptical, or as shown herein, having at a proximal portion thereof a flat base with rounded corners, from which a catenary arch shape (parabolic shape) extends distally. It is noted that while two frame elements are shown, other embodiments may include more than two frame elements in a partially overlapping arrangement (e.g., 3, 4, 5 frame elements).

A plurality of electrodes 315 are disposed on each of the frame elements, along the arched distal portion of the element. The electrodes are spaced from one another. The electrodes can be configured for ablation and/or sensing. Optionally, all electrodes are configured for both ablation and sensing.

In some examples, at least some of the electrodes are configured for ablation, and at least some of the electrodes (optionally, being electrodes of a different type) are configured for sensing.

In some examples, the electrodes of the two frame elements are staggered. In the collapsed state of the distal assembly, the electrodes fit in an alternating manner, thus enabling the distal assembly to fit within a small diameter of the sheath.

In some examples, a plurality of position sensors are disposed on the distal assembly and/or in or on the shaft. A position sensor can constitute one to three magnetic coils for sensing a position of at least a portion of the catheter, such as the distal assembly, along a corresponding one to three axes. In some embodiments, dual or triple axes position sensors (having two or three coils respectively) are located in device portions in which the volume is large enough to accommodate these sensors, for example inside the shaft; and in device portions in which the volume or mounting surface is limited, such as the distal assembly, single axis position sensors are used. Optionally, the coils of single axis sensors are printed coils.

In the example shown, a position sensor 329 constituting three magnetic coils for sensing a position in three axes is disposed or embedded within the shaft; and a plurality of single-coil position sensors 331 (for sensing a position along a single axis) are disposed on the frame elements, optionally, between adjacent electrodes, or optionally, mounted on the electrodes themselves.

In some examples, the frame elements are constructed from shape memory material, such as nitinol. Optionally, the frame is made of a nitinol wire. As such, when the distal assembly is advanced distally outwardly from the sheath (additionally or alternatively, the sheath is pulled proximally to allow release of the assembly), the assembly automatically springs back into its deployed state. The distal assembly is configured to assume its predetermined deployed state upon exiting the sheath, and remain in that configuration, thereby maintaining a spatial relationship between: the electrodes with respect to each other; the electrodes with respect to the positions sensors; the position sensors with respect to each other; the position sensors with respect to device portions, which can facilitate sensing and/or mapping. During use, the frame elements may slightly deform under externally applied force (such as force acting on the device when pressed against tissue), and return to their original shape once the force is removed.

In some examples, during the transition from the collapsed state to the deployed, expanded state, each of the frame element expands laterally with respect to a long axis of the frame element (see long axes 319, 321 respectively). Further, as shown in FIG. 3B, both of the frame elements curve such that the distal assembly assumes a concave (C-shaped) profile, configured to conform to the external curved surface of the epicardium. In some examples, the concavity is formed at a distal portion of the distal assembly, where the electrodes are disposed. For example, as shown, the curvature starts from a horizontal axis 323 and continues to the distal end of the distal assembly. In some examples, axis 323 is located at about 1/3, % or in any case at less than 1/2 of the length of the long axis of the frame element, such that the concavity is generally formed at a distal portion of the distal assembly, where the electrodes are disposed.

In use, the distal assembly is positioned with respect to the tissue such that the concavity faces the epicardium. This enables the electrodes to be placed in close contact with tissue which is spooned by the distal assembly.

In some examples, the frame elements partially overlap each other. The longitudinal axes 319 and 321 of the two frame elements can be spaced from one other. A potential advantage of multiple frame elements that partially overlap each other may include that a surface area covered by the distal assembly is relatively large, e.g., as compared to a single frame element or an arrangement in which the frame elements fully overlap. Covering a large tissue surface area may provide an improved therapeutic effect, as more tissue is simultaneously ablated; and may reduce the number of locations to which the catheter tip needs to be maneuvered, thereby potentially facilitating and accelerating the procedure. In some examples, wires of the two frame elements, at the overlapping portion, are interleaved with each other.

In some examples, the catheter comprises an inflatable element, such as a balloon 333 (a schematic representation thereof is shown). The balloon is configured to be inflated with fluid, such as saline, passed through the shaft or through a dedicated conduit, not shown. In some embodiments, in which irrigation fluid is supplied through the catheter, the balloon can be inflated with irrigation fluid.

The balloon is positioned with respect to the distal assembly such that when inflated, the electrodes of the distal assembly are pushed by the balloon towards the tissue surface. In some examples, the balloon is positioned and configured to serve as a back support layer to the distal assembly. Optionally, the balloon comes in contact with a surface of the electrodes which is opposite the energy emitting surface. A potential advantage of backing the distal assembly by a balloon (or other suitable inflatable element) may include enhancing the contact between the energy emitting surfaces of the electrodes and the tissue, and stabilizing the electrodes during sensing and/or ablation.

In some embodiments, the balloon is configured to be deployed from the distal end of the shaft along with the distal assembly. In some cases, the balloon is integral with the distal assembly and together they define a single deployable unit. Optionally, the balloon is mounted to the distal assembly at one or more portions thereof, for example at one or more locations of an electrode facing surface of the balloon.

In some examples, the balloon comprises one or more apertures 335 (see FIG. 3C) which allow for blood flow and/or irrigation flow into and/or out from the balloon. In some examples, the apertures define non-inflatable lumens, e.g., channels or slits, which extend across a volume of the balloon. Additionally or alternatively, apertures are in the form of small holes formed in the balloon material, which are sized to allow for controlled leakage of fluid. The rate in which fluid flows (e.g., drips slowly) through the holes is dictated, at least partially, by the size of the holes. In some cases, the small holes are located such that when the balloon is inflated, the holes are adjacent the electrodes and irrigation fluid supplied into the balloon can controllably leak through the holes to cool the electrodes, thereby potentially preventing overheating of the electrodes and/or overheating of adjacent non-target tissue.

In some examples, the balloon is formed as a unitary element; alternatively, the balloon is comprised of multiple elements, such as multiple elongate rib-like extensions which extend along at least a portion of the frame elements, at a side opposite that which contacts tissue.

In some examples, the balloon is positioned and configured to either produce or to deepen the concavity of the distal assembly, for example when being inflated inside the pericardial cavity such that the balloon presses on the distal assembly, causing it to conform to the rounded tissue surface. In such case, the distal assembly can be flat (planar) in its neutral state, and be at least partially deformed by the balloon when inflated.

Referring back to the electrodes, the electrodes are mounted on the frame elements such that in the deployed, expanded state of the distal assembly, energy emitting surfaces 316 (or generally, tissue contacting surfaces) of the electrodes face a similar direction, towards a center of the concavity. Optionally and preferably, the energy emitting surfaces of the electrodes of both frame elements face a similar direction.

In some examples, portions of an electrode other than a portion facing towards a center of the concavity are covered with an insulating material, which reduces or prevents the transfer of energy and/or heat. Additionally or alternatively to an insulating cover, the inflated balloon can be positioned at a back side of the electrode or spline and electrically and/or thermally isolate the backside of the electrode. As such, the balloon may reduce or prevent unintended energy emission from the electrode in the direction of the pericardium sac.

Typically, the electrodes are ring shaped, yet other electrode shapes (e.g., flat electrodes, roller shaped electrodes, hexagonal or other shapes) are also contemplated.

In some examples, the catheter comprises one or more means for irrigation. In some examples, the frame elements can be formed with irrigation outlets (e.g., holes formed by puncturing, drilling, laser cutting or as such; not shown) and irrigation is supplied via the shaft and then through the frame elements, exiting via the irrigation outlets. Additionally or alternatively, irrigation outlets (not shown) are provided in the electrodes, e.g. by a hole that extends through the electrode, and irrigation is supplied via the shaft, through the frame elements, exiting via the irrigation outlets of the electrodes. Additionally or alternatively, an irrigation tube and/or spray sleeve (not shown) are provided, for example extending distally from the shaft or surrounding the shaft.

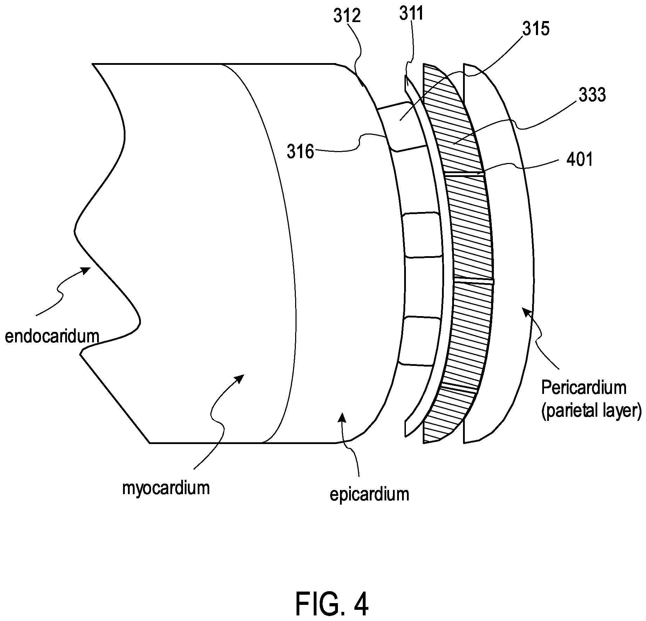

**FIG. 4** is a schematic illustration of a portion of the distal tip of the catheter shown in FIGs. 3A-B, placed in contact with epicardial tissue, according to examples of the presently disclosed subject matter.

The schematic illustration shows a curved portion of the frame element 311 which is arched to fit about a generally curved surface 312 of the epicardium. The electrodes 315 are disposed along the curve, with their energy emitting surfaces 316 facing and contacting the epicardium surface. As can be observed, the curvature of the frame elements, in this example, is generally C-shaped.

In some examples, as shown, balloon 333 is inflated on a back (convex) side of the frame element, for exerting pressure onto the frame element and specifically onto the electrodes to ensure that the energy emitting/receiving surfaces of the electrodes closely contact the tissue. Optionally, the balloon is inflated to exert pressure onto the electrodes such that the electrodes are slightly forced against the tissue. In some examples, as shown, the balloon is inflated inside the pericardial cavity, between the epicardium and the parietal layer of the pericardium. As the balloon is inflated in this confined space, the parietal layer of the pericardium compresses the balloon and provides a counter force which may contribute to having the other side of the balloon press against the electrodes.

Further shown herein, according to some examples, are a plurality of apertures, for example formed as channels 401 that extend throughout the balloon. The channels can remain free of inflation fluid and allow irrigation fluid and/or blood to flow through. In some examples, the balloon comprises separately inflatable cells which are divided from each other by the channels. Alternatively, the balloon comprises a single inflatable cell.

In some examples, the balloon is shaped similar to the frame elements of the distal assembly, or at least to their outermost contour, forming a lining to the frame elements at their back side.

In use, electrodes 315 serve for sensing electrical cardiac activity from the surface of the epicardium, and/or for delivering ablation energy to the tissue. The energy can be applied in a bipolar mode, between pairs of electrodes on the assembly, or in a unipolar mode, for example between the electrodes and a separate common electrode. The common electrode may be configured within a back patch for placing onto the patient's skin. Additionally or alternatively, a common electrode can be a reference electrode located on or in the catheter itself.

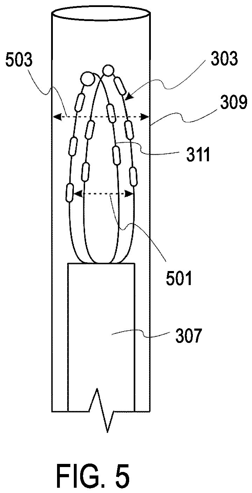

**FIG. 5** is a schematic cross section view of the distal tip of the catheter shown in FIGs. 3A-B, showing the distal assembly in the collapsed state inside a sheath of the catheter, according to examples of the presently disclosed subject matter.

In the shown example, the distal assembly 303 is in a collapsed state, in which the distal assembly is configured to fit inside the lumen of sheath 309. In the collapsed state, a maximal diameter 501 of the distal assembly is at least 5%, 10%, 15% shorter than a diameter 503 of the sheath. The distal assembly is maintained in its collapsed state inside the sheath during, for example, introducing of the catheter into the body, during transfer of the catheter between treatment locations, during removal of the catheter from the body.

In some examples, as shown, in the collapsed state the frame elements 311 are maintained close together. The wire frame of each element is brought closer to the long axis of the element, and the electrodes 315 are staggered. In some examples, the collapsed state is obtained by pulling the distal assembly (optionally, by pulling on shaft 307) proximally to pull the distal assembly into the sheath; additionally or alternatively, the sheath is pushed distally to surround the distal assembly and compress it into its lumen.

In other examples, the frame elements of the distal assembly can be rolled or otherwise folded about a long axis of the shaft.

### EXAMPLES OF THE PRESENT DISCLOSURE

### Example 1

A catheter for mapping and ablation of epicardial tissue, comprising:
a shaft configured for insertion into a body of a subject;
a distal assembly configured at a distal end of the shaft, the distal assembly being expandable from a collapsed state to a deployed state;
the distal assembly comprising at least two frame elements; each frame element comprising an arched distal portion on which a plurality of electrodes for delivery of ablation energy are disposed; wherein in the deployed state, the distal assembly assumes a concave profile for conforming to a curvature of the epicardium; and
a plurality of position sensors disposed on one or both of the shaft and the frame elements for tracking a position and an orientation of at least a portion of the distal assembly or the shaft.

### Example 2

The catheter according to Example 1, further comprising an inflatable element extendable from the distal end of the shaft and positioned, when inflated, to back the distal assembly and apply pressure onto the distal assembly from a side opposite a tissue contacting side of the distal assembly, so as to push the plurality of electrodes of the distal assembly against the tissue.

### Example 3

The catheter according to Example 1 or Example 2, wherein each of the plurality of electrodes comprises an energy emitting surface configured to contact the tissue, and wherein the plurality of electrodes are disposed on the frame elements such that their energy emitting surfaces are on a tissue contacting side of the distal assembly.

### Example 4

The catheter according to Example 3, wherein the energy emitting surfaces of the plurality of electrodes face a center of the concavity defined by the distal assembly.

### Example 5

The catheter according to any one of Examples 1-4, wherein the plurality of electrodes are staggered with respect to each other so that in the collapsed state of the distal assembly the electrodes alternately fit adjacent one another.

### Example 6

The catheter according to any one of Examples 1-5, wherein in the deployed state of the distal assembly the at least two frame elements at least partially overlap.

### Example 7

The catheter according to any one of Examples 1-6, wherein each of the frame elements defines a closed contour with the distal end of the shaft.

### Example 8

The catheter according to any one of Examples 1-7, wherein each of the frame elements is comprised of shape memory material.

### Example 9

The catheter according to any one of Examples 1-8, wherein the plurality of electrodes are configured to deliver radiofrequency (RF) ablation energy or pulsed-field ablation (PFA) energy.

### Example 10

The catheter according to any one of Examples 1-9, wherein the plurality of electrodes are configured for sensing cardiac electrical activity.

### Example 11

The catheter according to any one of Examples 1-10, further comprising an external sheath in which the shaft and the distal assembly, when in the collapsed state, are contained; and wherein the distal assembly is configured to expand from the collapsed state to the deployed state upon protruding from a distal end of the sheath.

### Example 12

The catheter according to any one of Examples 1-11, wherein the plurality of position sensors include one or more of: a single axis position sensor, a dual axis position sensor, and a triple axis position sensor, the position sensors being operable with a magnetic pad of a position sensing system.

### Example 13

The catheter according to any one of Examples 2-12, wherein the inflatable element comprises a plurality of apertures through which fluid is allowed to flow.

### Example 14

The catheter according to any one of Examples 2-13, wherein the inflatable element comprises a single inflatable cell, or multiple inflatable cells.

### Example 15

A method of ablating epicardial tissue, comprising:
introducing a catheter comprising, at a distal tip thereof, a distal assembly configured to assume a concave profile for conforming to a curvature of the epicardial tissue;
inflating an inflatable element on a back side of the distal assembly; and
delivering ablation energy via the electrodes to the epicardial tissue.

### Example 16

The method according to Example 15, wherein the inflatable element is inflated between the epicardium and the parietal layer of the pericardium.

### Example 17

The method according to Example 15 or Example 16, wherein the catheter further comprises a plurality of position sensors and wherein said method comprises tracking a position and an orientation of at least a portion of the catheter using the position sensors.

### Example 18

The method according to any one of Examples 15-17, further comprising sensing cardiac electrical activity via electrodes of the distal assembly.

### Example 19

The method according to Example 18, comprising identifying, based on the sensed cardiac electrical activity, arrhythmic patterns associated with an epicardial source.

Those skilled in the art to which the present invention pertains, can appreciate that while the present invention has been described in terms of preferred examples, the concept upon which this disclosure is based may readily be utilized as a basis for the designing of other structures, systems and processes for carrying out the several purposes of the present invention.

The various illustrative logical blocks, modules, and algorithm steps described in connection with the examples disclosed herein can be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing any departure from the scope of the disclosure.

It will also be understood that the system according to the present disclosure may be, at least partly, implemented on a suitably programmed computer. Likewise, the present disclosure contemplates a computer program being readable by a computer for executing the method of the invention. The present disclosure further contemplates a non-transitory computer-readable memory tangibly embodying a program of instructions executable by the computer for executing the method of the present disclosure.

Also, it is to be understood that the phraseology and terminology employed herein are for the purpose of description and should not be regarded as limiting.

It should be noted that the words "comprising", "including" and "having" as used throughout the appended claims are to be interpreted to mean "including but not limited to". The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases, and disjunctively present in other cases.

It is important, therefore, that the scope of the invention is not construed as being limited by the illustrative examples set forth herein. Other variations are possible within the scope of the present invention as defined in the appended claims. Other combinations and sub-combinations of features, functions, elements and/or properties may be claimed through amendment of the present claims or presentation of new claims in this or a related application. Such amended or new claims, whether they are directed to different combinations or directed to the same combinations, whether different, broader, narrower or equal in scope to the original claims, are also regarded as included within the subject matter of the present description.

## Claims

1. A catheter for mapping and ablation of epicardial tissue, comprising:
a shaft configured for insertion into a body of a subject;
a distal assembly configured at a distal end of the shaft, the distal assembly being expandable from a collapsed state to a deployed state;
the distal assembly comprising at least two frame elements; each frame element comprising an arched distal portion on which a plurality of electrodes for delivery of ablation energy are disposed; wherein in the deployed state, the distal assembly assumes a concave profile for conforming to a curvature of the epicardium; and
a plurality of position sensors disposed on one or both of the shaft and the frame elements for tracking a position and an orientation of at least a portion of the distal assembly or the shaft.

2. The catheter according to claim 1, further comprising an inflatable element extendable from the distal end of the shaft and positioned, when inflated, to back the distal assembly and apply pressure onto the distal assembly from a side opposite a tissue contacting side of the distal assembly, so as to push the plurality of electrodes of the distal assembly against the tissue.

3. The catheter according to claim 1 or claim 2, wherein each of the plurality of electrodes comprises an energy emitting surface configured to contact the tissue, and wherein the plurality of electrodes are disposed on the frame elements such that their energy emitting surfaces are on a tissue contacting side of the distal assembly.

4. The catheter according to claim 3, wherein the energy emitting surfaces of the plurality of electrodes face a center of the concavity defined by the distal assembly.

5. The catheter according to any preceding claim, wherein the plurality of electrodes are staggered with respect to each other so that in the collapsed state of the distal assembly the electrodes alternately fit adjacent one another.

6. The catheter according to any preceding claim, wherein in the deployed state of the distal assembly the at least two frame elements at least partially overlap.

7. The catheter according to any preceding claim, wherein each of the frame elements defines a closed contour with the distal end of the shaft.

8. The catheter according to any preceding claim, wherein each of the frame elements is comprised of shape memory material.

9. The catheter according to any preceding claim, wherein the plurality of electrodes are configured to deliver radiofrequency (RF) ablation energy or pulsed-field ablation (PFA) energy.

10. The catheter according to any preceding claim, wherein the plurality of electrodes are configured for sensing cardiac electrical activity.

11. The catheter according to any preceding claim, further comprising an external sheath in which the shaft and the distal assembly, when in the collapsed state, are contained; and wherein the distal assembly is configured to expand from the collapsed state to the deployed state upon protruding from a distal end of the sheath.

12. The catheter according to any preceding claim, wherein the plurality of position sensors include one or more of: a single axis position sensor, a dual axis position sensor, and a triple axis position sensor, the position sensors being operable with a magnetic pad of a position sensing system.

13. The catheter according to any of claims 2 to 12, wherein the inflatable element comprises a plurality of apertures through which fluid is allowed to flow.

14. The catheter according to any of claims 2 to 13, wherein the inflatable element comprises a single inflatable cell, or multiple inflatable cells.
